(19) Europäisches Patentamt
European Patent Office
Office européen des brevets

(11) Veröffentlichungsnummer : **0 573 387 A2**

(12) # EUROPÄISCHE PATENTANMELDUNG

(21) Anmeldenummer : **93810368.6**

(22) Anmeldetag : **19.05.93**

(51) Int. Cl.$^5$ : **C07D 217/10,** C07D 261/04, C07D 237/26, C07D 237/08, C07D 241/46, C08K 5/29, H01B 1/12

(30) Priorität : **03.06.92 CH 1775/92**

(43) Veröffentlichungstag der Anmeldung : **08.12.93 Patentblatt 93/49**

(84) Benannte Vertragsstaaten : **AT BE CH DE ES FR GB IT LI NL SE**

(71) Anmelder : **CIBA-GEIGY AG**
**Klybeckstrasse 141**
**CH-4002 Basel (CH)**

(72) Erfinder : **Chetcuti, Peter, Dr.**
**Münchensteinerstrasse 8**
**CH-4052 Basel (CH)**

(54) **Charge-Transfer Komplexe mit N-aromatischen Verbindungen, deren Herstellung und deren Verwendung.**

(57) Charge-Transfer Komplexe der Formel I

$$(A^{\ominus})_n B^{n\oplus} \quad \text{(I)},$$

worin
a) n 1 oder 2 ist,
b) A das Radikalanion einer Verbindung der Formel II oder einer Mischung von Verbindungen der Formel II ist,

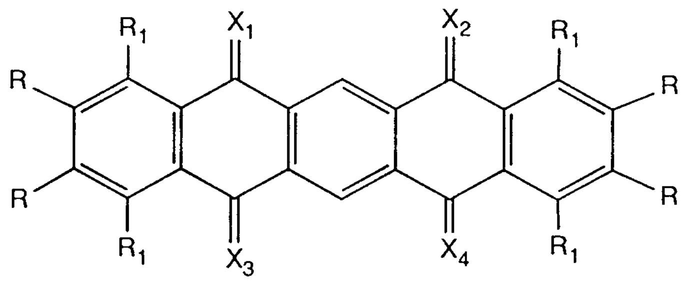

(II),

worin die R gleich sind und für H oder $C_1$-$C_4$-Alkyl stehen, oder die benachbarten R zusammen $-(CH_2)_3-$ oder $-(CH_2)_4-$darstellen ; $R_1$ H oder $C_1$-$C_4$-Alkyl bedeutet ; und $X_1$ =N-CN darstellt und $X_2$, $X_3$ und $X_4$ =O oder =N-CN bedeuten, und
c) falls n = 1, B das einwertige Radikalkation, und falls n = 2, B das zweiwertige Radikalkation einer N-aromatischen Verbindung mit insgesamt 1 bis 5 unsubstituierten oder mit Halogen, $C_1$-$C_4$-Alkyl oder $C_1$-$C_4$-Alkoxy substituierten aromatischen Ringen ist, wobei mindestens ein Ring mindestens eine Gruppe $-NR_2-$ oder $[=N^+R_2-]\ I^-$ enthält, worin $R_2$ $C_1$-$C_4$-Alkyl oder Benzyl ist.
Die Komplexe sind elektrische Leiter, mit denen Kunststoffe antistatisch ausgerüstet oder in elektrische Leiter übergeführt werden können.

EP 0 573 387 A2

Die Erfindung betrifft Charge-Transfer Komplexe (nachfolgend als CT-Komplexe abgekürzt) aus Pentacencyanoiminderivaten als Elektronenakzeptoren und Stickstoff-aromatischen Verbindungen als Elektronendonatoren; ein Verfahren zu deren Herstellung; Zusammensetzungen aus einem Kunststoff und einem solchen CT-Komplex; und die Verwendung der CT-Komplexe als elektrische Leiter, zum Beispiel zur Herstellung von elektrisch leitenden Filmen, Folien oder Beschichtungen.

In Synthetic Metals, 41-43, Seiten 2365 bis 2375 (1991) wird ein pulverförmiger CT-Komplex aus Tetracyanoiminpentacen und Tetrathiofulvalen als Elektronendonator, sowie Einzelkristalle aus Tetracyanoiminpentacen und Alkalimetallkationen und Tetraalkylammonium beschrieben.

Ferner wird 5,7,12,14-Tetracyanoiminpentacen von L. Miller et al. in Chem. Mater. 2, Seiten 339-40 (1990) als Elektronenakzeptor zur Herstellung von Radikalkationensalzen mit Alkalimetallen wie zum Beispiel Natrium und Kalium beschrieben.

Im US Patent 5,009,812 werden antistatisch ausgerüstete und elektrisch leitfähige Polymere beschrieben, die zum Beispiel CT-Komplexe aus Tetrathio-, Tetraseleno- oder Tetratellurotetracenen als Elektronendonatoren und Halogenen oder Sauerstoff als Elektronenakzeptoren enthalten. Die CT-Komplexe bilden in diesen Materialien Nadelnetzwerke in der Polymermatrix.

CT-Komplexe aus Tetracyanochinodimethan (TCNQ) als Elektronenakzeptor und N-enthaltenden aromatischen Verbindungen als Donatoren sind, z.B. von C. D. Jaeger und A. J. Bard in J. Am. Chem. Soc., Vol.102, Nr. 17, Seiten 5435-5442 (1980), und von L. Russell Melby in Can. J. Chem., Vol. 43, Seiten 1448-1453 (1965) beschrieben. Diese CT-Komplexe kristallisieren nicht immer nadelförmig, und sind auf Grund ihrer Kristallgestalt nicht zur Herstellung von elektrisch leitenden Folien mit einem Netzwerk aus Kristallnadeln geeignet.

Es wurde nun gefunden, dass Pentacencyanoimine und bestimmte N-aromatische Verbindungen überraschend CT-Komplexe bilden, die unerwartet selbst in Gegenwart von Bindemitteln nadelförmig kristallisieren, eine hohe elektrische Leitfähigkeit aufweisen und praktisch keine korrosive Wirkung auf metallische Teile von Verarbeitungsmaschinen zeigen. Die Ausgangsverbindungen sind auch in weniger polaren organischen Lösungsmitteln löslich, so dass zur Herstellung der CT-Komplexe keine sehr hohen Temperaturen angewendet werden müssen. Die CT-Komplexe weisen eine unerwartet hohe Stabilität gegenüber Feuchtigkeit und Wärme auf. Ferner bilden die CT-Komplexe überraschend feine und stabile Kristallnadeln aus, wodurch Filme oder Folien mit sehr feinmaschigen Nadelnetzwerken und hoher elektrischer Leitfähigkeit erhalten werden.

Ein Gegenstand der Erfindung sind CT-Komplexe der Formel I

$$(A^{\ominus})_n B^{n\oplus} \quad \text{(I)},$$

worin

a) n 1 oder 2 ist,

b) A das Radikalanion einer Verbindung der Formel II oder einer Mischung von Verbindungen der Formel II ist,

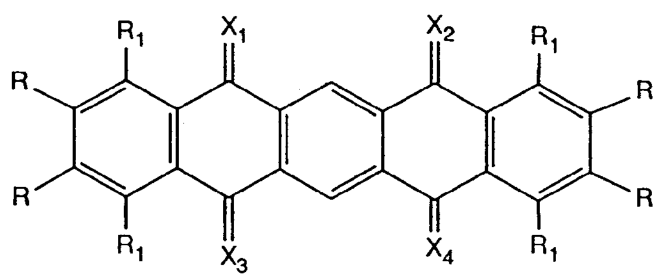

(II),

worin die R gleich sind und für H oder $C_1$-$C_4$-Alkyl stehen, oder die benachbarten R zusammen $-(CH_2)_3-$ oder $-(CH_2)_4-$ darstellen; $R_1$ H oder $C_1$-$C_4$-Alkyl bedeutet; und $X_1$ =N-CN darstellt und $X_2$, $X_3$ und $X_4$ =O oder =N-CN bedeuten, und

c) falls n = 1, B das einwertige Radikalkation, und falls n = 2, B das zweiwertige Radikalkation einer N-aromatischen Verbindung mit insgesamt 1 bis 5 unsubstituierten oder mit Halogen, $C_1$-$C_4$-Alkyl oder $C_1$-$C_4$-Alkoxy substituierten aromatischen Ringen ist, wobei mindestens ein Ring mindestens eine Gruppe $-NR_2-$ oder $[=N^+R_2-]\ I^-$ enthält, worin $R_2$ $C_1$-$C_4$-Alkyl oder Benzyl ist.

Bei R und $R_1$ in der Bedeutung von Alkyl kann es sich um Methyl, Ethyl, n- oder i-Propyl oder n-, i- oder t-Butyl handeln. Bevorzugte Alkylreste sind Methyl und Ethyl. In einer bevorzugten Ausführungsform stellen die R $C_1$-$C_4$-Alkyl und die $R_1$ H dar, oder stellen die $R_1$ $C_1$-$C_4$-Alkyl und die R H dar. Bevorzugt stellen R und $R_1$ H, Methyl oder Ethyl dar. In einer besonders bevorzugten Ausführungsform bedeuten R und $R_1$ H.

In einer anderen bevorzugten Ausführungsform bedeuten $X_1$ und $X_4$ =N-CN und $X_2$ und $X_3$ =O oder =N-

CN, oder $X_2$ und $X_3$ =N-CN und $X_4$ =O oder =N-CN. Besonders bevorzugt stellen $X_1$, $X_2$, $X_3$ und $X_4$ =N-CN dar.

Das Alkyl kann linear oder verzweigt sein. Beispiele sind Methyl, Ethyl, n- oder i-Propyl, und n-, i- oder t-Butyl. Bevorzugt ist $R_2$ Methyl oder Ethyl; besonders bevorzugt ist $R_2$ Methyl.

Bei den ein- oder zweiwertigen Radikalkationen B kann es sich um N-heterocyclische Ringe mit einem oder zwei N-Atomen handeln.

B enthält bevorzugt insgesamt 1 bis 3 und mindestens einen N-aromatischen Ring. In einer bevorzugten Ausführungsform enthält B 1 bis 3 Ringe und einen heteroaromatischen Ring oder B stellt einen bis-N-heteroaromatischen Ring dar. Die Ringe sind bevorzugt 6-gliedrig. Besonders bevorzugte N-Aromaten sind Pyridin, Pyrimidin, Pyrazin und Phenazin.

In einer besonders bevorzugten Ausführungsform entspricht B in Formel I Kationen der Formeln IIIa bis IIIf:

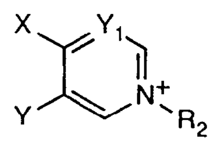 , 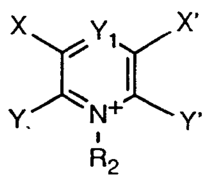 , 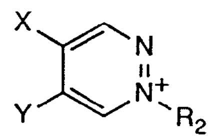 ,

IIIa IIIb IIIc

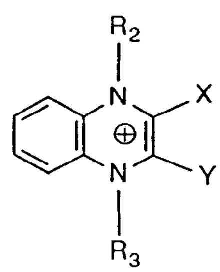 , 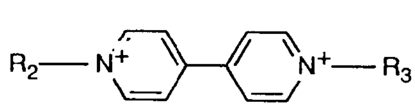 , 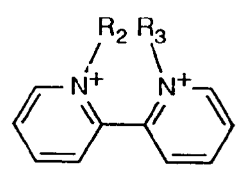 ,

IIId IIIe IIIf

worin $Y_1$ N oder CH bedeutet, und X, X', Y und Y' für H stehen oder X und Y und/oder X' und Y' die Gruppe -CH=CH-CH=CH- bedeuten, und $R_3$ unabhängig die gleiche Bedeutung wie $R_2$ hat.

Bei B in Formel I kann es sich zum Beispiel um Kationen von N-Methyl- und N-Ethyl-pyridinium; N-Methyl- und N-Ethyl-pyrazinium; N-Methyl- und N-Ethyl-chinolinium; N-Methyl- und N-Ethyl-phthalazinium; N-Methyl und N-Ethyl-isochinolinium; N-Methyl- und N-Ethyl-benzopyrazinium; 4,4'-Dimethyl-, 4,4'-Diethyl- und 4-Methyl,4'-ethyl-bipyridinium; N-Methyl- und N-Ethyl-acridinium; N-Methyl- und N-Ethyl-phenazinium; 2,2'-Dimethyl-, 2,2'-Diethyl- und 2-Methyl,2'-ethyl-bipyridinium; N-Methyl- oder N-Ethylpyridazinium; 5,10-Dimethyl-, 5,10-Diethyl- oder 5-Methyl,10-ethyl-5,10-dihydrophenazinium handeln.

Die Verbindung der Formel II ist bevorzugt 5,7,12,14-Tetracyanoiminpentacen, das in reiner Form vorliegt, oder bis zu 10 Gew-%, bezogen auf die Gesamtmischung, Verbindungen der Formel II enthält, in denen ein oder zwei Cyanoimingruppen durch Sauerstoff ersetzt sind. Besonders bevorzugte CT-Komplexe der Formel I sind solche aus 5,7,12,14-Tetracyanoiminpentacen und N-Methylpyrazinium, N-Ethylpyrazinium, 5,10-Dimethyl-5,10-dihydrophenazinium, N-Methylchinolinium, N-Methylisochinolinium oder N-Methylbenzopyridazinium als B.

Ein anderer Gegenstand der Erfindung ist ein Verfahren zur Herstellung von CT-Komplexen der Formel I, das dadurch gekennzeichnet ist, dass man 1 Äquivalent eines neutralen 5,10-Dihydrophenazinderivats oder dessen Iodsalz als B oder das Iodsalz einer N-aromatischen Verbindung B mit mindestens 1 Äquivalent eines Pentacencyanoimins der Formel II in einem inerten organischen Lösungsmittel umsetzt. Es kann aber zweckmässig sein, einen Überschuss des Pentacencyanoimins zu verwenden.

Die N-aromatischen Verbindungen B sind als Salze oder freie Basen bekannt, teilweise käuflich oder sie können nach allgemein bekannten Verfahren hergestellt werden. Reaktion zwischen der neutralen Base B und

dem Alkyliodid ergibt das gewünschte Iodsalz. Die Herstellung von einigen von diesen Derivaten ist von L. Russell Melby in Can. J. Chem. Vol. 43, Seiten 1448 bis 1453 (1965) beschrieben.

Die Herstellung von 5,7,12,14-Tetracyanoiminpentacen ist von L. L. Miller in Synthetic Metals, 41-43, Seiten 2365-2375 (1991) beschrieben. Die als Ausgangsverbindungen verwendeten unsubstituierten oder substituierten 5,7,12,14-Pentacentetrone sind nach einem von W. H. Mills et al. in J. Chem. Soc. 101, Seite 2194 (1912) beschriebenen Verfahren erhältlich. Die 5,7,12,14-Tetracyanoiminpentacene können nach üblichen Methoden gereinigt werden, zum Beispiel durch Umkristallisation oder chromatographische Verfahren. Wenn keine besonderen Schutzmassnahmen ergriffen werden, wie zum Beispiel wasserfreie Bedingungen, können Cyanoimingruppen durch Sauerstoff ersetzt werden, was aber die Bildung der gewünschten CT-Komplexe nicht negativ beeinflusst.

Das erfindungsgemässe Verfahren wird vorteilhaft bei erhöhten Temperaturen durchgeführt, zum Beispiel bei Raumtemperatur bis 150°C. Zur Isolierung der erfindungsgemässen CT-Komplexe kann man das Reaktionsgemisch abkühlen und die ausgefallenen Kristalle abfiltrieren und durch auswaschen und/oder Umkristallisation reinigen.

Geeignete Lösungsmittel sind zum Beispiel unpolare, polare und aprotische Lösungsmittel, die allein oder in Mischungen aus mindestens zwei Lösungsmitteln verwendet werden können. Beispiele sind: Ether (Anisol, Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1 ,2,2-Tetrachlorethan), Carbonsäureester und Lactone (Essigsäureethylester, Propionsäuremethylester, Benzoesäureethylester, 2-Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Ketone (Methylethylketon, Methylisobutylketon), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol), Nitrile (Acetonitril, Propionitril) und aliphatische oder cycloaliphatische Kohlenwasserstoffe (Petrolether, Pentan, Hexan, Cyclohexan und Methylcyclohexan). Geeignet sind auch aromatisch-aliphatische Ether wie zum Beispiel Methyl- oder Ethylphenylether.

Bevorzugt sind polare Lösungsmittel, weil die Iodsalze der Verbindungen B unter diesen Bedingungen besser löslich sind. Bevorzugte polare Lösungsmittel sind zum Beispiel Dimethylformamid und γ-Butyrolacton.

Die nach dem erfindungsgemässen Verfahren erhältlichen CT-Komplexe fallen in hohen Reinheiten an und brauchen nach der Filtration nur noch mit Lösungsmitteln gewaschen werden. Es handelt sich in der Regel um dunkelfarbige Kristalle mit nadelförmiger Kristallgestalt, die elektrische Leitfahigkeiten als Pressling von mehr als $1 \cdot 10^{-4}$ S/cm aufweisen. Sie eignen sich hervorragend als elektrische Leiter. So können mit der Einarbeitung dieser CT-Komplexe in Kunststoffe je nach Art des CT-Komplexes und der verwendeten Menge elektrisch leitende oder antistatisch ausgerüstete Kunststoffe erhalten werden, wobei der CT-Komplex als Netzwerk der Kristallnadeln in der Kunststoffmatrix vorliegt. Je nach der Konzentration des CT-Komplexes in der Kunststoffmatrix können sehr feinmaschige Nadelnetzwerke erhalten werden.

Ein weiterer Gegenstand der Erfindung ist eine Zusammensetzung, enthaltend a) ein duroplastisches, thermoplastisches oder strukturell vernetztes Polymer und b) einen CT-Komplex der Formel I in Form eines Netzwerks aus Kristallnadeln in der Polymermatrix.

Die CT-Komplexe können in einer Menge von 0,01 bis 30 Gew.-%, bevorzugt 0,01 bis 20 Gew.-%, besonders bevorzugt 0,01 bis 10 Gew.-% und insbesondere bevorzugt 0,1 bis 5 Gew.-% enthalten sein, bezogen auf die Zusammensetzung.

Bei den thermoplastischen Polymeren kann es sich zum Beispiel um die folgenden Polymeren, Copolymeren bzw. Mischungen von diesen Polymeren handeln:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzahigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylendiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamid-systeme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Polyether aus Diglycidylverbindungen, zum Beispiel Diglycidylethern und Diolen, zum Beispiel aus Bisphenol-A-Diglycidylether und Bisphenol-A.

21. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

22. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Bevorzugte Thermoplaste sind Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol.

Bei den Duroplasten und strukturell vernetzten Polymeren kann es sich zum Beispiel um folgende Polymere handeln:

1. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

2. Trocknende und nicht-trocknende Alkydharze.

3. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

4. Vernetzbare Acrylharze, die sich von substituierten Acrylsäureestern ableiten, wie z.B. von Epoxyacrylaten, Urethan-acrylaten oder Polyester-acrylaten.

5. Alkydharze, Polyesterharze und Acrylatharze, die mit Melaminharzen, Harnstoffharzen, Polyisocyanaten oder Epoxidharzen vernetzt sind.

6. Kautschuk auf der Basis von vernetzten Polydienen, zum Beispiel Butadien oder Isopren; Silikonkautschuk.

7. Epoxidharze, die sich von Polyepoxiden ableiten, z.B. von Bisglycidylethern von Polyolen oder von cycloaliphatischen Diepoxiden, und die gegebenenfalls einen Härter als Vernetzungsmittel enthalten, oder die unter Verwendung von Härtungsbeschleunigern thermisch oder durch Einwirkung von Strahlung vernetzt sind.

Unter den vernetzten Polymeren sind vernetzte Epoxidharze bevorzugt, denen als Polyepoxide bevorzugt Glycidylverbindungen mit durchschnittlich zwei Epoxidgruppen im Molekül zu Grunde liegen. Als Glycidylverbindungen kommen vor allem solche mit zwei an ein Heteroatom (z.B. Schwefel, vorzugsweise Sauerstoff oder Stickstoff) gebundenen Glycidylgruppen, β-Methylglycidylgruppen oder 2,3-Epoxycyclopentylgruppen in Frage; genannt seien insbesondere Bis-(2,3-epoxycyclopentyl)-ether, Diglycidylether von mehrwertigen aliphatischen Alkoholen, wie 1,4-Butandiol, oder Polyalkylenglykolen, wie Polypropylenglykole; Diglycidylether von cycloaliphatischen Polyolen, wie 2,2-Bis-(4-hydroxycyclohexyl)-propan; Diglycidylether von mehrwertigen Phenolen, wie Resorcin, Bis-(p-hydroxyphenyl)-methan, 2,2-Bis-(p-hydroxyphenyl)-propan (=Diomethan), 2,2-Bis-(4'-hydroxy-3',5'-dibromphenyl)-propan, 1,3-Di-(p-hydroxyphenyl)-ethan; Di-(β-methylglycidyl)-ether der oben angeführten zweiwertigen Alkohole oder zweiwertigen Phenole; Diglycidylester von Dicarbonsäuren, wie Phthalsäure, Terephthalsäure, $\Delta_4$-Tetrahydrophthalsäure und Hexahydrophthalsäure; N,N-Diglycidylderivate von primären Aminen und Amiden und heterocyclischen, zwei N-Atome enthaltenden Stickstoffbasen, und N,N'-Diglycidylderivate von disekundären Diamiden und Diaminen, wie N,N-Diglycidylanilin, N,N-Diglycidyltoluidin, N,N-Diglycidyl-p-aminophenyl-methyl-ether, N,N'-Dimethyl-N,N'-diglycidyl-bis-(p-aminophenyl)-methan; N',N''-Diglycidyl-N-phenyl-isocyanurat; N,N'-Diglycidylethylenharnstoff; N,N'-Diglycidyl-5,5-dimethylhydantoin, N,N'-Diglycidyl-5-isopropyl-hydantoin, N,N-Methylen-bis-(N',N'-diglycidyl-5,5-dimethylhydantoin), 1,3-Bis-(N-glycidyl-5,5-dimethylhydantoin)-2-hydroxypropan; N,N'-Diglycidyl-5,5-dimethyl-6-isopropyl-5,6-dihydro-uracil, Triglycidylisocyanurat.

Eine bevorzugte Gruppe von Epoxidharzen sind glycidylierte Novolake, Hydantoine, Aminophenole, Bisphenole und aromatische Diamine oder cycloaliphatische Epoxidverbindungen. Besonders bevorzugte Epoxidharze sind glycidylierte Kresolnovolake, Bisphenol-A- und Bisphenol-F-diglycidylether, Hydantoin-N,N'-bisglycid, p-Aminophenoltriglycid, Diaminodiphenylmethantetraglycid, Vinylcyclohexendioxid, 3,4-Epoxycyclohexylmethyl-3,4-epoxycyclohexancarboxylat oder Mischungen hiervon.

Geeignet sind auch vorreagierte Addukte solcher Epoxidverbindungen mit Epoxidhärtern, zum Beispiel ein Addukt aus Bisphenol-A-diglycidylether und Bisphenol-A, oder mit Oligoestern mit zwei terminalen Carboxylgruppen und Epoxiden vorreagierte Addukte.

Als Härter für Epoxidharze kommen säure oder basische Verbindungen in Frage. Als geeignete Härter seien zum Beispiel genannt: mehrwertige Phenole (Resorcin, 2,2-Bis-(4-hydroxyphenyl)-propan) oder Phenol-Formaldehyd-Harze; mehrbasische Carbonsäuren und ihre Anhydride, z. B. Phthalsäureanhydrid, Tetrahydrophthalsäureanhydrid, Hexahydrophthalsäureanhydrid, 4-Methylhexahydrophthalsäureanhydrid, 3,6-Endomethylen-tetrahydrophthalsäreanhydrid, 4-Methyl-3,6-endomethylen-tetrahydrophthalsaureanhydrid (Methylnadicanhydrid), 3,4,5,6,7,7-Hexachlor-endomethylen-tetrahydrophthalsäureanhydrid, Bernsteinsäureanhydrid, Adipinsäureanhydrid, Trimethyladipinsäureanhydrid, Sebacinsäureanhydrid, Maleinsäureanhydrid, Dodecylbernsteinsäureanhydrid, Pyromellitsäuredianhydrid, Trimellitsäureanhydrid, Benzophenontetracarbonsäuredianhydrid, oder Gemische solcher Anhydride.

Eine bevorzugte Gruppe von Härtern sind Novolake und Polycarbonsäureanhydride.

Die Epoxidharze können auch zusätzlich mit Härtungsbeschleunigern oder nur mit thermischen Härtungskatalysatoren gehärtet werden. Beispiele sind 3-Ethyl-4-methylimidazol, Triamylammoniumphenolat; Mono- oder Polyphenole (Phenol, Diomenthan, Salicylsäure); Bortrifluorid und seine Komplexe mit organischen Verbindungen wie z.B. Bortrifluorid-Etherkomplexe und BortrifluoridAmin-Komplexe ($BF_3$-Monoethylamin-Komplex); Phosphorsäure und Triphenylphosphit.

Härtungsbeschleuniger und Katalysatoren werden üblicherweise in einer Menge von 0,1 bis 10 Gew.-% zugegeben, bezogen auf das Epoxidharz. Härter für Epoxidharze werden im allgemeinen in äquimolaren Mengen verwendet, bezogen auf die Epoxidgruppen und funktionellen Gruppen eines Härters.

Der erfindungsgemässen Zusammensetzung können weitere Additive zur Verbesserung der Verarbeitungseigenschaften, der mechanischen, elektrischen und thermischen Eigenschaften, der Oberflächeneigenschaften und der Lichtstabilität einverleibt sein, zum Beispiel feinteilige Füllstoffe, Verstärkerfüllstoffe, Weichmacher, Gleit- und Entformungsmittel, Haftvermittler, Antistatika, Antioxidantien, Wärme- und Lichtstabilisatoren, Pigmente und Farbstoffe.

Eine bevorzugte Ausführungsform der erfindungsgemässen Zusammensetzung ist dadurch gekennzeichnet, dass sie als Formkörper, Film, Folie, Faser, oder als Beschichtung auf mindestens einer Oberfläche eines Substrats ausgebildet ist.

Ein weiterer Gegenstand der Erfindung ist ein Verfahren zur Herstellung von erfindungsgemässen Zusammensetzungen, das dadurch gekennzeichnet ist, dass man (a) einen CT-Komplex der Formel I einem thermoplastischen Kunststoff einverleibt, (b) einen CT-Komplex der Formel I mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel II, ein 5,10-Dihydrophenazinderivat oder dessen Iodsalz als B oder das Iodsalz einer N-aromatischen Verbindung B zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung eines 5,10-Dihydrophenazinderivats oder dessen Iodsalz als B oder eines Iodsalzes einer N-aromatischen Verbindung B beziehungsweise einer Verbindung der Formel II vermischt, das Lösungsmittel entfernt und härtbare Mischungen zu einem duroplastischen oder strukturell vernetzten Kunststoff polymerisiert. Das Herstellungsverfahren kann mit einer Formgebung verbunden sein.

Die Herstellung der erfindungsgemässen Zusammensetzung kann nach in der Kunststofftechnik bekannten Verfahren erfolgen. Bei Formgebungsverfahren für Polymere, zum Beispiel Giessen, Pressverfahren, Spritzgiessen und Extrusion, kann der CT-Komplex selbst unter Bildung von Suspensionen einem Thermoplasten oder mindestens einem Ausgangsstoff für Duroplaste, oder getrennt je einem Ausgangsstoff (zum Beispiel dem Epoxidharz und dem Härter) unter Bildung von Lösungen oder Suspensionen zugegeben werden, wobei nach der Formgebung der CT-Komplex während des Abkühlens in Form von Nadeln auskristallisiert beziehungsweise ausfällt, und die Nadeln ein Netzwerk in einer Polymermatrix bilden.

In einer besonders bevorzugten Ausführungsform ist die erfindungsgemässe Zusammensetzung als Film oder Folie oder als Beschichtung auf mindestens einer Oberfläche eines Substrats ausgebildet. Zur Herstellung solcher Ausführungsformen wird zum Beispiel ein thermoplastisches Polymer oder mindestens ein Ausgangsprodukt für einen Duroplasten oder ein strukturell vernetztes Polymer in einem inerten Lösungsmittel zusammen mit einem CT-Komplex der Formel I suspendiert und /oder gelöst, oder zusammen mit einer Verbindung der Formel II oder einem Iodsalz einer N-aromatischen Verbindung B gelöst und dann mit einer Lösung

des N-aromatischen Iodsalzes B beziehungsweise einer Verbindung der Formel II versetzt und vermischt, danach mittels bekannter Beschichtungstechniken auf ein gegebenenfalls vorerwärmtes Substrat aufgetragen und dann unter Erwärmen das Lösungsmittel entfernt, wobei vernetzbare Mischungen dann noch ausgehärtet werden können. Freitragende Filme und Folien werden durch Ablösen vom Substrat oder mittels Extrusionsverfahren erhalten.

Geeignete Substrate sind zum Beispiel Glas, Metalle, Kunststoffe, mineralische und keramische Werkstoffe, Holz und Papier. Die Substrate können beliebige äussere Formen aufweisen; es kann sich zum Beispiel um Formkörper, Fäden, Fasern, Gewebe, Stangen, Rohre, Bänder, Folien, Platten, Walzen oder Gehäuse handeln.

Geeignete Beschichtungsverfahren sind zum Beispiel Streichen, Walzen, Rakeln, Giessen, Schleudergiessen, Vorhanggiessen und Sprayen. Besonders bevorzugte Verfahren sind Sprayverfahren, weil zum einen sehr dünne und gleichmässige Schichten mit im wesentlichen isotropen, sehr feinmaschigen und homogenen Netzwerken aus Kristallnadeln der CT-Komplexe erhältlich sind, und zum anderen die Grösse der Kristallnadeln und die Maschenweite der Netzwerke durch die Tröpfchengrösse kontrolliert werden kann, selbst wenn man Suspensionen sprüht.

Geeignete inerte Lösungsmittel für Polymere beziehungsweise Ausgangsprodukte für Polymere sind zum Beispiel polare und bevorzugt aprotische Lösungsmittel, die alleine oder in Mischungen aus mindestens zwei Lösungsmitteln verwendet werden können. Beispiele sind: Ether (Anisol, Dibutylether, Tetrahydrofuran, Dioxan, Ethylenglykolmonomethyl- oder -dimethylether, Ethylenglykolmonoethyl- oder -diethylether, Diethylenglykoldiethylether, Triethylenglykoldimethylether), halogenierte Kohlenwasserstoffe (Methylenchlorid, Chloroform, 1,2-Dichlorethan, 1,1,1-Trichlorethan, 1,1,2,2-Tetrachlorethan), Carbonsäureester und Lactone (Essigsäureethylester, Propionsäuremethylester, Benzoesäureethylester, 2Methoxyethylacetat, γ-Butyrolacton, δ-Valerolacton, Pivalolacton), Carbonsäureamide und Lactame (N,N-Dimethylformamid, N,N-Diethylformamid, N,N-Dimethylacetamid, Tetramethylharnstoff, Hexamethylphosphorsäuretriamid, γ-Butyrolactam, ε-Caprolactam, N-Methylpyrrolidon, N-Acetylpyrrolidon, N-Methylcaprolactam), Ketone (Methylethylketon, Methylisobutylketon), Sulfoxide (Dimethylsulfoxid), Sulfone (Dimethylsulfon, Diethylsulfon, Trimethylensulfon, Tetramethylensulfon), tertiäre Amine (N-Methylpiperidin, N-Methylmorpholin) substituierte Benzole (Benzonitril, Chlorbenzol, o-Dichlorbenzol, 1,2,4-Trichlorbenzol, Nitrobenzol, Toluol, Xylol) und Nitrile (Acetonitril, Propionitril). Geeignet sind auch aromatisch-aliphatische Ether wie zum Beispiel Methyl- oder Ethylphenylether. Geeignete Lösungsmittel für die Verbindungen der Formel II und die N-aromatischen Verbindungen/Iodsalze B sind zuvor genannt worden.

Die Beschichtungsverfahren können zum Beispiel so durchgeführt werden, dass man die einzelnen Bestandteile getrennt löst und erst vor dem gewählten Beschichtungsverfahren vereinigt. Man kann aber auch die Bestandteile in zwei Lösungen, zum Beispiel Polymerlösung und N-aromatisches Iodsalz B oder einer Verbindung der Formel II und Lösung einer Verbindung der Formel II oder eines N-aromatischen Iodsalzes B gegebenenfalls zusammen mit einem Polymer herstellen, oder alle Bestandteile in einer Lösung vereinigen. Im letzten Fall können die CT-Komplexe schon vor der Beschichtung auskristallisieren, was aber praktisch keinen Einfluss auf die gewünschte Qualität der Beschichtung hat.

Die Lösungen werden zweckmässig erwärmt, zum Beispiel auf 30 bis 200 °C. Es ist vorteilhaft, auch das Substrat zu erwärmen, um die Entfernung des Lösungsmittels zu beschleunigen, die im allgemeinen bei Temperaturen von 50 bis 150 °C, bevorzugt 50 bis 100 °C vorgenommen wird, bis die Beschichtung trocken ist. Sofern die Beschichtungen zu frei tragenden Filmen oder Folien abgelöst werden sollen, kann das Substrat vor der Beschichtung mit Antihaftmitteln behandelt werden.

In einer Herstellvariante für Beschichtungen kann man auch die erfindungsgemässen CT-Komplexe, die als Kristallnadeln ausgebildet sind, in einer Lösung eines Polymeren oder Ausgangsstoffen für Duroplaste suspendieren, dann ein Substrat beschichten und danach das Lösungsmittel entfernen und gegebenenfalls zur Bildung der Duroplaste aushärten. Ferner ist es möglich, pulvrige Trockenmischungen aus Polymerpulvern oder festen Ausgangsstoffen für Duroplaste und den CT-Komplexen herzustellen und diese in gegebenenfalls elektrostatischen Beschichtungsverfahren zu Schichten auf Substraten zu verarbeiten. Auch bei diesen Varianten werden Netzwerke von Kristallnadeln in einer Polymermatrix erhalten.

Es ist auch möglich, reine Schichten von Netzwerken aus Kristallnadeln der CT-Komplexe auf einem Substrat herzustellen, indem man zum Beispiel Lösungen oder Suspensionen der CT-Komplexe in einem Lösungsmittel auf einem Substrat aufbringt und danach das Lösungsmittel verdampft. Solche Schichten können zur Erhöhung der elektrischen Leitfähigkeit elektrochemisch metallisiert werden, zum Beispiel mit Cu, Pt oder Pd. Es kann zweckmässig sein, solche reine Schichten mit einer Schutzschicht aus einem Polymer zu beschichten oder die reinen Schichten nachträglich mit einem Polymer zu umhüllen.

Die Schichtdicken können in einem breiten Rahmen variieren, je nach Wahl des Beschichtungsverfahrens. Mit Sprayverfahren können sehr dünne Schichten erhalten werden, während man mit Streich- und

Giessverfahren auch dicke Schichten erzielen kann. Die Schichtdicken können zum Beispiel 0,01 bis 5000 μm, bevorzugt 0,1 bis 1000 μm und besonders bevorzugt 0,1 bis 500 μm betragen.

Die erfindungsgemässe Zusammensetzung ist je nach Wahl des Polymers opak oder transparent und sie weist hervorragende elektrische Eigenschaften auf. So weisen die Beschichtungen und Formkörper überraschend eine ausgezeichnete Entladungsfähigkeit auf, die für heterogene Materialien sonst schwierig oder gar nicht zu erreichen ist. Die Zusammensetzung eignet sich daher besonders zur Verwendung als antistatisch ausgerüstete Formteile für die elektrostatische Abschirmung von Bauteilen oder zur Verwendung als antistatisch ausgerüstete Formkörper. Die hohen elektrischen Leitfähigkeiten ermöglichen ferner die Verwendung als elektrische Leiter, zum Beispiel als Elektroden für Displayelemente oder elektronische Bauteile sowie als Ladungsspeicher in Kondensatoren. Die Zusammensetzungen weisen auch hervorragende mechanische Festigkeiten und mechanische Gebrauchseigenschaften auf. Die Zusammensetzungen können auch bei vergleichsweise niedrigen Temperaturen hergestellt werden und bieten zudem den Vorteil, keine oder nur eine unwesentliche Korrosion bei metallischen Maschinenteilen zu verursachen. Ferner weisen sie gute Stabilitäten gegenüber dem Einfluss von Wärme und/oder Feuchtigkeit auf.

Weitere Gegenstände der Erfindung sind die Verwendung der erfindungsgemässen Charge-Transfer Komplexe der Formel I als elektrische Leiter; die Verwendung der erfindungsgemässen Zusammensetzung als antistatisch ausgerüstete Formteile für die elektrostatische Abschirmung von Bauteilen oder als antistatisch ausgerüstete Formkörper; die Verwendung der erfindungsgemässen Zusammensetzung als elektrische Leiter; die Verwendung der erfindungsgemässen Zusammensetzung als Elektrodenmaterial und die Verwendung der erfindungsgemässen Zusammensetzung in Form von Filmen oder Folien als Ladungsträger in Kondensatoren.

Die nachfolgenden Beispiele erläutern die Erfindung näher.

A) Herstellungsbeispiele

Beispiel A1: Herstellung von N-Methylisochinolinium.5,7,12,14-tetracyanoiminpentacen.

Zu einer Lösung von 100 mg (0,369 mMol) N-Methylisochinoliniumiodid in 40 ml Dimethylformamid (DMF) wird bei Raumtemperatur eine gleich warme filtrierte Lösung von 160 mg (0,369 mMol) 5,7,12,14-Tetracyanoiminpentacen in 60 ml DMF gegeben. Man erhält eine dunkelgrüne Lösung, die man 15 Stunden bei 5°C stehen lässt. Danach werden die ausgefallenen braunen Kristallnadeln abfiltriert, mit DMF und Diethylether gewaschen und dann im Vakuum getrocknet. Man erhält 66,7 mg (31 % Ausbeute) der Titelverbindung, deren elektrische Leitfähigkeit (gemessen nach der Vierpunktmethode an einem Pressling) 0,034 S/cm beträgt. Elementaranalyse gefunden (berechnet) für $C_{36}H_{20}N_9$.DMF: C 71,67 (71,88); H 4,22 (4,18); N 21,35 (21,49). Zersetzungstemperatur 271°C.

Beispiel A2: Herstellung von N-Methylchinolinium. 5,7,12,14-tetracyanoiminpentacen.

Zu einer Lösung von 100 mg (0,369 mMol) N-Methylchinoliniumiodid in 20 ml DMF wird bei Raumtemperatur eine filtrierte Lösung von 160 mg (0,369 mMol) 5,7,12,14-Tetracyanoiminpentacen in 30 ml DMF gegeben. Man erhält eine grüne Lösung, die man 15 Stunden bei 5°C stehen lässt. Danach werden die ausgefallenen braunen Kristallnadeln abfiltriert, mit DMF und Diethylether gewaschen und im Vakuum getrocknet. Man erhält 110 mg (52 % Ausbeute) der Titelverbindung. Die elektrische Leitfähigkeit (Pressling) beträgt 0,16 S/cm. Elementaranalyse gefunden (berechnet) für $C_{36}H_{20}N_9$: C 74,55 (74,73); H 3,58 (3,48); N 21,78 (21,79). Zersetzungstemperatur 266°C.

Beispiel A3: Herstellung von N-Methylbenzopvridazinium.5,7,12,14-tetracyanoiminpentacen.

Zu einer Lösung von 100 mg (0,367 mMol) N-Methylbenzopyridaziniumiodid in 10 ml γ-Butyrolacton, die eine Temperatur von 100°C aufweist, wird eine gleich warme Lösung von 159 mg (0,367 mMol) 5,7,12,14-Tetracyanoiminpentacen in 50 ml DMF gegeben. Man lässt die Lösung bis Raumtemperatur abkühlen und 15 Stunden bei 5°C stehen. Danach werden die ausgefallenen grün-schwarzen Kristallnadeln abfiltriert, mit Diethylether gewaschen und im Vakuum getrocknet. Man erhält 213 mg (100 % Ausbeute) der Titelverbindung. Die elektrische Leitfähigkeit (Pressling) beträgt 1,85 S/cm. Elementaranalyse gefunden (berechnet) für $C_{35}H_{19}N_{10}$: C 71,81 (72,53); H 2,98 (3,30); N 24,73 (24,17). Zersetzungstemperatur 273°C.

Beispiel A4: Herstellung von N-Methylpyrazinium.5,7,12,14-tetracyanoiminpentacen.

Zu einer Lösung von 77 mg (0,345 mMol) N-Methylpyraziniumiodid in 30 ml DMF, die eine Temperatur von 100°C aufweist, wird eine gleich warme Lösung von 150 mg (0,345 mMol) 5,7,12,14-Tetracyanoiminpentacen in 50 ml DMF gegeben. Man erhält eine dunkelrote Lösung, die nach Abkühlung bis Raumtemperatur eine dunkelgrüne kristalline Suspension ergibt. Man erhält nach Abfiltrieren und Waschen mit Diethylether 57 mg, und es kristallisieren aus dem Filtrat weitere 42 mg der Titelverbindung: insgesamt 99 mg (54 % Ausbeute) feine grüne Kristallnadeln. Die elektrische Leitfähigkeit (Pressling) beträgt 2,40 S/cm. Elementaranalyse gefunden (berechnet) für $C_{31}H_{17}N_{10}$: C 70,28 (70,31); H 2,87 (3,24); N 26,08 (26,45). Zersetzungstemperatur 240°C.

Beispiel A5: Herstellung von 5,10-Dimethyl-5,10-dihydrophenazinium.5,7,12,14-tetracyanoiminpentacen.

Zu einer Lösung von 100 mg (0,476 mMol) 5,10-Dimethyl-5,10-dihydrophenazin in 20 ml Anisol, die eine Temperatur von 110°C aufweist, wird eine gleich warme Lösung von 207 mg (0,476 mMol) 5,7,12,14-Tetracyanoiminpentacen in 70 ml Anisol gegeben. Man erhält eine orange Lösung, die nach Abkühlung bis Raumtemperatur 15 Stunden steht. Danach werden die ausgefallenen braunen Kristallnadeln abfiltriert, mit Anisol und Dichlorethan gewaschen und im Vakuum getrocknet. Man erhält 210 mg (68 % Ausbeute) der Titelverbindung. Die elektrische Leitfähigkeit (Pressling) beträgt 0,082 S/cm. Elementaranalyse gefunden (berechnet) für $C_{40}H_{24}N_{10}$: C 74,46 (74,52); H 4,01 (3,75); N 20,37 (21,73). Zersetzungstemperatur 297°C.

B) Anwendungsbeispiel

Beispiel B1

Zu einer 130 °C warmen Lösung von 200 mg Polycarbonat und 2,1 mg (0,01 mMol) 5,10-Dimethyl-5,10-dihydrophenazin in 7 ml Anisol wird eine 130 °C warme Lösung von 4,5 mg 5,7,12,14-Tetracyanoiminpentacen in 7 ml Anisol zugegeben. Aliquote Teile (je 2 ml) werden auf eine Glasplatte gegossen und dann das Lösungsmittel bei verschiedenen Temperaturen abgedampft. An den erhaltenen Folien wird anschliessend die Leitfähigkeiten gemessen.

| Abdampftemperatur (°C) | Leitfähigkeit (S/cm) |
|---|---|
| 100 | $2,3 \cdot 10^{-4}$ |
| 110 | $2,1 \cdot 10^{-4}$ |

**Patentansprüche**

1. Charge-Transfer Komplexe der Formel I

$$(A^{\ominus})_n B^{n\oplus} \quad (I),$$

worin

a) n 1 oder 2 ist,

b) A das Radikalanion einer Verbindung der Formel II oder einer Mischung von Verbindungen der Formel II ist,

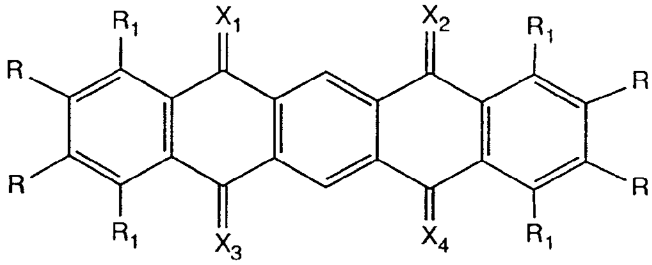

(II),

worin die R gleich sind und für H oder $C_1$-$C_4$-Alkyl stehen, oder die benachbarten R zusammen $-(CH_2)_3-$ oder $-(CH_2)_4-$ darstellen; $R_1$ H oder $C_1$-$C_4$-Alkyl bedeutet; und $X_1$ =N-CN darstellt und $X_2$, $X_3$ und $X_4$ =O oder =N-CN bedeuten, und

c) falls n = 1, B das einwertige Radikalkation, und falls n = 2, B das zweiwertige Radikalkation einer N-aromatischen Verbindung mit insgesamt 1 bis 5 unsubstituierten oder mit Halogen, $C_1$-$C_4$-Alkyl oder $C_1$-$C_4$-Alkoxy substituierten aromatischen Ringen ist, wobei mindestens ein Ring mindestens eine Gruppe $-NR_2-$ oder $[=N^+R_2-]I^-$ enthält, worin $R_2$ $C_1$-$C_4$-Alkyl oder Benzyl ist.

**2.** Komplexe gemäss Anspruch 1, dadurch gekennzeichnet, dass R $C_1$-$C_4$-Alkyl bedeutet und $R_1$ für H steht.

**3.** Komplexe gemäss Anspruch 1, dadurch gekennzeichnet, dass $R_1$ $C_1$-$C_4$-Alkyl bedeutet und R für H steht.

**4.** Komplexe gemäss Anspruch 1, dadurch gekennzeichnet, dass R und $R_1$ in der Bedeutung von Alkyl für Methyl oder Ethyl stehen.

**5.** Komplexe gemäss Anspruch 1, dadurch gekennzeichnet, dass R und $R_1$ für H, Methyl oder Ethyl stehen.

**6.** Komplexe gemäss Anspruch 1, dadurch gekennzeichnet, dass R und $R_1$ für H stehen.

**7.** Komplexe gemäss Anspruch 1, dadurch gekennzeichnet, dass $X_1$ und $X_4$ =N-CN und $X_2$ und $X_3$ =O oder =N-CN, oder $X_2$ und $X_3$ =N-CN und $X_4$ =O oder =N-CN.

**8.** Komplexe gemäss Anspruch 1, dadurch gekennzeichnet, dass $X_1$, $X_2$, $X_3$ und $X_4$ =N-CN darstellen.

**9.** Komplexe gemäss Anspruch 1, dadurch gekennzeichnet, dass N-heterocyclische Ringe des Kations B 1 oder 2 N-Atome enthalten.

**10.** Komplexe gemäss Anspruch 1, dadurch gekennzeichnet, dass B in Formel I Kationen der Formeln IIIa bis IIIf entspricht:

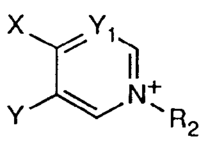 ,
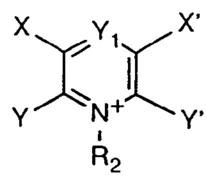 ,
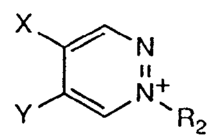 ,

IIIa IIIb IIIc

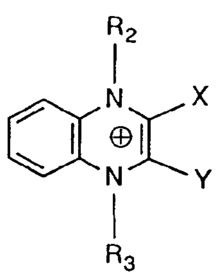 ,
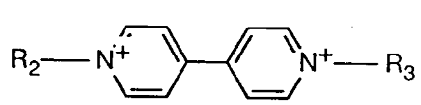 ,
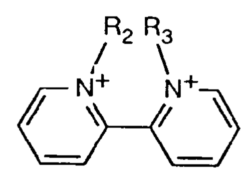 ,

IIId IIIe IIIf

worin $Y_1$ N oder CH bedeutet, und X, X', Y und Y' für H stehen oder X und Y und/oder X' und Y' die Gruppe -CH=CH-CH=CH- bedeuten, und $R_3$ unabhängig die gleiche Bedeutung wie $R_2$ hat.

**11.** Komplexe gemäss Anspruch 10, dadurch gekennzeichnet, dass $R_2$ und $R_3$ Methyl oder Ethyl bedeuten.

12. Komplexe gemäss Anspruch 10, dadurch gekennzeichnet, dass es sich bei B um Kationen von N-Methyl- oder N-Ethyl-pyridinium; N-Methyl- oder N-Ethyl-pyrazinium; N-Methyl- oder N-Ethyl-chinolinium; N-Methyl- oder N-Ethyl-phthalazinium; N-Methyl oder N-Ethyl-isochinolinium; N-Methyl- oder N-Ethyl-benzopyrazinium; 4,4'-Dimethyl-, 4,4'-Diethyl- oder 4-Methyl,4'-ethyl-bipyridinium; N-Methyl- oder N-Ethylacridinium; N-Methyl- oder N-Ethyl-phenazinium; 2,2'-Dimethyl-, 2,2'-Diethyl- oder 2-Methyl,2'-ethyl-bipyridinium; N-Methyl- oder N-Ethylpyridazinium; 5,10-Dimethyl-, 5,10-Diethyl- oder 5-Methyl,10-ethyl-5,10-dihydrophenazinium handelt.

13. Komplexe gemäss Anspruch 1, dadurch gekennzeichnet, dass in Formel I A für 5,7,12,14-Tetracyanoiminpentacen steht und B N-Methylpyrazinium, N-Ethylpyrazinium, 5,10-Dimethyl-5,10-dihydrophenazinium, N-Methylchinolinium, N-Methylisochinolinium oder N-Methylbenzopyridazinium ist.

14. Verfahren zur Herstellung von Charge-Transfer Komplexen der Formel I gemäss Anspruch 1, dadurch gekennzeichnet, dass man 1 Äquivalent eines neutralen 5,10-Dihydrophenazinderivats oder dessen Iodsalz als B oder das Iodsalz einer N-aromatischen Verbindung B mit mindestens 1 Äquivalent eines Pentacencyanoimins der Formel II in einem inerten organischen Lösungsmittel umsetzt.

15. Zusammensetzung, enthaltend a) ein duroplastisches, thermoplastisches oder strukturell vernetztes Polymer und b) einen Charge-Transfer Komplex der Formel I gemäss Anspruch 1 in Form eines Netzwerks aus Kristallnadeln in der Polymermatrix.

16. Zusammensetzung gemäss Anspruch 15, dadurch gekennzeichnet, dass der Charge-Transfer Komplex in einer Menge von 0,01 bis 30 Gew.-% enthalten ist, bezogen auf die Zusammensetzung.

17. Zusammensetzung gemäss Anspruch 16, dadurch gekennzeichnet, dass der Charge-Transfer Komplex in einer Menge von 0,01 bis 10 Gew.-% enthalten ist.

18. Zusammensetzung gemäss Anspruch 15, dadurch gekennzeichnet, dass es sich bei dem thermoplastischen Polymer um Polyolefine, Polystyrol, Polyvinylchlorid, Polyvinylidenchlorid, Polyvinylidenfluorid, Polyacrylate, Polymethacrylate, Polyamide, Polyester, Polycarbonate, aromatische Polysulfone, aromatische Polyether, aromatische Polyethersulfone, Polyimide und Polyvinylcarbazol handelt.

19. Zusammensetzung gemäss Anspruch 15, dadurch gekennzeichnet, dass es sich bei dem duroplastischen Polymer um ein Epoxidharz handelt.

20. Zusammensetzung gemäss Anspruch 15, dadurch gekennzeichnet, dass sie als Formkörper, Film, Folie, Faser oder als Beschichtung auf mindestens einer Oberfläche eines Substrats ausgebildet ist.

21. Zusammensetzung gemäss Anspruch 20, dadurch gekennzeichnet, dass die Schichtdikke der Beschichtungen 0.01 bis 5000 μm beträgt.

22. Zusammensetzung gemäss Anspruch 20, dadurch gekennzeichnet, dass die Schichtdik-ke der Beschichtungen 0,1 bis 1000 μm beträgt.

23. Verfahren zur Herstellung von Zusammensetzungen gemäss Anspruch 15, dadurch gekennzeichnet, dass man (a) einen CT-Komplex der Formel I einem thermoplastischen Kunststoff einverleibt, (b) einen CT-Komplex der Formel I mindestens einer Komponente eines duroplastischen oder strukturell vernetzbaren Kunststoffs einverleibt und dann die Mischung gegebenenfalls zusammen mit weiteren Komponenten zu einem duroplastischen oder strukturell vernetzten Kunststoff polymerisiert, oder (c) eine Verbindung der Formel II oder ein 5,10-Dihydrophenazinderivat oder dessen Iodsalz als B oder ein Iodsalz der N-aromatischen Verbindung B zusammen mit einem thermoplastischen Kunststoff oder mindestens einer Komponente eines duroplastischen oder strukturell vernetzbaren Kunststoffs in einem organischen Lösungsmittel löst, diese Lösung gegebenenfalls zusammen mit weiteren Komponenten für einen duroplastischen oder strukturell vernetzbaren Kunststoff mit einer Lösung eines 5,10-Dihydrophenazinderivats oder dessen Iodsalz als B oder des Iodsalzes einer N-aromatischen Verbindung B beziehungsweise einer Verbindung der Formel II vermischt, das Lösungsmittel entfernt und härtbare Mischungen zu einem duroplastischen oder strukturell vernetzten Kunststoff polymerisiert.

24. Verfahren gemäss Anspruch 23, dadurch gekennzeichnet, dass es mit einer Formgebung verbunden ist.

**25.** Verwendung der Charge-Transfer Komplexe der Formel I gemäss Anspruch 1 als elektrische Leiter.

**26.** Verwendung der Zusammensetzung gemäss Anspruch 15 als antistatisch ausgerüstete Formteile für die elektrostatische Abschirmung von Bauteilen oder als antistatisch ausgerüstete Formkörper.

**27.** Verwendung der Zusammensetzung gemäss Anspruch 15 als elektrische Leiter.

**28.** Verwendung der Zusammensetzung gemäss Anspruch 15 als Elektrodenmaterial.

**29.** Verwendung der Zusammensetzung gemäss Anspruch 15 in Form von Filmen oder Folien als Ladungsträger in Kondensatoren.